(19) Europäisches Patentamt
European Patent Office
Office européen des brevets

(11) Veröffentlichungsnummer: **0 082 339**
**B1**

(12) # EUROPÄISCHE PATENTSCHRIFT

(45) Veröffentlichungstag der Patentschrift: **16.07.86**

(51) Int. Cl.⁴: **C 07 D 239/47**

(21) Anmeldenummer: **82110854.5**

(22) Anmeldetag: **24.11.82**

(54) **Verfahren zur Herstellung von Cytosin.**

(30) Priorität: **23.12.81 DE 3150929**

(43) Veröffentlichungstag der Anmeldung: **29.06.83 Patentblatt 83/26**

(45) Bekanntmachung des Hinweises auf die Patenterteilung: **16.07.86 Patentblatt 86/29**

(84) Benannte Vertragsstaaten: **AT BE CH DE FR GB IT LI LU NL SE**

(56) Entgegenhaltungen:
**US - A - 2 892 840**

**JOURNAL OF THE CHEMICAL SOCIETY, 1965, Seite 1515, Nr. 268, London, G.B., J.A. HILL et al.: "The isolation of a Hitherto undescribed sulphate of cytosine and an improved preparation of the intermediate 1-cyano-2,2-diethoxy-ethane"**
**J. biol. Chem. 177 (1949), 565**
**J. org. Chem. 22 (1957) 22**
**C.A. 59 (1963) 6510**

**Die Akte enthält technische Angaben, die nach dem Eingang der Anmeldung eingereicht wurden und die nicht in dieser Patentschrift enthalten sind.**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel 3 (DE)**
Erfinder: **Theis, Christoph, Dr., Hoher Rain 5, D-5216 Niederkassel-Rheidt (DE)**
Erfinder: **Vogt, Wilhelm, Dr., Kleiststrasse 39, D-5000 Köln 40 (DE)**

**EP 0 082 339 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Cytosin (3) aus 3-Alkoxyacrylnitril (1) und/oder 3,3-Dialkoxypropionitril (2) und Harnstoff in Gegenwart einer Base und anschliessender Neutralisation entsprechend dem Reaktionsschema:

$R^1O-CH=CH-CN$ (1)

oder

$(R^2O)_2\ CH-CH_2-CN$ (2)

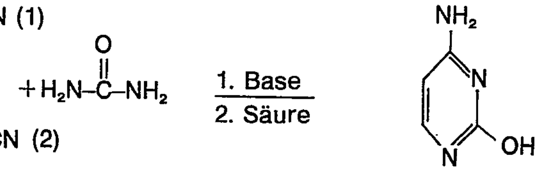


(3)

Bei Reaktionen dieser Ausgangsstoffe wird nach dem Stand der Technik stets zunächst Cytosinsulfat oder -hemisulfat erhalten, nicht jedoch freies Cytosin, das nur unter hohem Aufwand und erheblichen Verlusten aus den Sulfaten gewonnen werden kann: (J. biol. Chem. 177 (1949) 565; J. org. Chem. 22 (1957) 192; Brit. Pat. 806 235; C.A. 59 (1963) 6510; J. Chem. Soc. (1965) 1515). Dabei wird 3-Ethoxyacrylnitril oder Cyanacetaldehyddiethylacetal mit Harnstoff in Gegenwart von Natrium-n-butylat unter Rückflusssieden zu einem nicht definierten Vorprodukt des Cytosins umgesetzt, das nach einem sehr aufwendigen Verfahren durch Zugabe von überschüssiger, verdünnter wässriger Schwefelsäure in das Sulfat oder Hemisulfat des Cytosins überführt und nur durch Zugabe von viel Ethanol als Cytosinsulfat ausfällt und abgetrennt werden kann. Nach Auflösen des isolierten Cytosinsulfats in Wasser wird das freie Cytosin durch Zugabe von Ammoniak ausgefällt.

Diese Verfahrensweise hat schwerwiegende Nachteile: Zur Gewinnung des Cytosins und zur Rückgewinnung der eingesetzten Lösungsmittel sind eine Vielzahl von zum Teil aufwendigen Verfahrensschritten notwendig. Das schliesslich erhaltene Cytosin fällt stark verunreinigt mit Cytosinsulfat an und kann auf Grund ähnlicher Löslichkeitseigenschaften nur äusserst schwer und mit grossen Verlusten von Cytosinsulfat getrennt werden. Die Bildung des Cytosin-Heterocyclus erfolgt gemäss dem Stand der Technik erst in Gegenwart eines Überschusses an Schwefelsäure, die zur Ausfällung des freien Cytosins wieder neutralisiert werden muss, wodurch erhebliche Mengen Schwefelsäuren und Ammoniak verbraucht werden, entsprechende Salze anfallen und entsorgt werden müssen.

Das Cytosinsulfat kann aus der wässrigen Lösung nur durch Zugabe eines grossen Ethanolüberschusses ausgefällt werden. Nachteilig sind die grossen Lösungsmittelmengen und deren kostspielige, mit Verlusten verbundene Aufarbeitung.

Aufgabe der vorliegenden Erfindung ist die Herstellung von Cytosin in hoher Ausbeute und Reinheit unter Vermeidung grosser Mengen von Lösungsmitteln und Neutralisationsmitteln während der umständlichen Fällungs-, Kristallisations- und Neutralisationsvorgänge. Eine weitere Aufgabe bestand in der Verwendung eines Gemisches von 3-Alkoxyacrylnitril und 3,3-Dialkoxypropionitril, das als Verunreinigung 3-Alkoxy-2-alkylacrylnitril und 3,3-Dialkoxy-2-alkylpropionitril enthält.

Es wurde überraschend gefunden, dass Cytosin direkt als freie Verbindung erhalten werden kann. Die Bildung eines Cytosinsalzes, dessen Kristallisation, Gewinnung, Auflösung und Neutralisation mit Basen kann vermieden werden, wenn erfindungsgemäss Essigsäure, Ameisensäure, Chlorwasserstoff oder Phosphorsäure in zur Neutralisation ausreichender Menge nach der Reaktion zugegeben werden.

Gegenstand der Erfindung ist ein Verfahren zur Herstellung von Cytosin, welches dadurch gekennzeichnet ist, dass man 3-Alkoxyacrylnitril (1) oder 3,3-Dialkoxypropionitril (2) oder Gemische hiervon als Nitrilkomponente, Harnstoff und Alkali- oder Erdalkalialkoholate bzw. -Hydroxide als Base nach dem Reaktionsschema

$R^1O-CH=CH-CN$ (1)

oder

$(R^2O)_2\ CH-CH_2-CN$ (2)

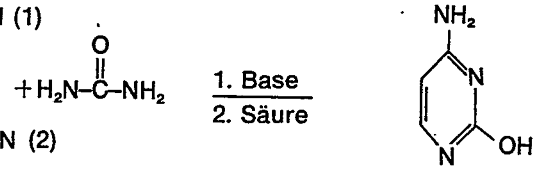


(3)

worin $R^1$ die Bedeutung geradkettige oder verzweigte Alkyl- oder Alkenylreste mit 1 oder 12 C-Atomen, isocyclische oder heterocyclische, einkernige oder mehrkernige aromatische oder cycloaliphatische Ringsysteme, welche gegebenenfalls Substituenten tragen, oder $-(CH_2)_n$-Cyc mit Cyc = isocyclische oder herocyclische, einkernige oder mehrkernige aromatische oder cycloaliphatische Ringsysteme, welche gegebenenfalls Substituenten an den Ringen tragen, und n = 0 bis 5 ist, die Reste $(CH_2)_mOR'$ oder $-(CH_2CH_2O)_p OR'$ mit m = 1 bis 5 und p = 1 bis 4 und R' = geradkettige oder verzweigte Alkylreste mit 1 bis 12 C-Atomen, und $R^2$ dieselbe Bedeutung wie $R^1$ oder die Bedeutung eines Alkylen oder Alkylen-Restes eines Ringes mit 3 bis 6 Gliedern, der ggf. durch ein oder mehrere Heteroatome unterbrochen ist, hat, zur Reaktion bringt und zur Aufarbeitung nach beendeter Reaktion das Reaktionsgemisch durch Zugabe von Essigsäure, Ameisensäure, Chlorwasserstoff oder Phosphorsäure neutralisiert und freies Cytosin abscheidet und gewinnt.

Ein Gemisch von 3-Alkoxyacrylnitril und 3,3-Dialkoxypropionitril, das als Verunreinigung 3-Alkoxy-2-alkylacrylnitril und 3,3-Dialkoxy-2-alkylpropionitril enthält, kann zur Synthese von reinem Cytosin eingesetzt werden. Überraschend wurde weiter gefunden, dass durch Dosierung des 3-Alkoxyacrylnitrils oder 3,3-Dialkoxypropionitrils oder des genannten Gemisches zu den übrigen Reaktanden, besonders bei Reaktionstemperatur, die Ausbeute gegenüber dem beschriebenen Verfahren erhöht wird.

Die als Ausgangsstoff verwendeten 3-Alkoxyacrylnitrile (1) sind nach der DE-OS 2 912 345 gut zugänglich. Bei dieser Synthese fällt zunächst ein Rohgemisch von 3-Alkoxyacrylnitril (1) (O-Alkylierung) und 3-Alkoxy-2-alkylacrylnitril (O- und C-Alkylierung) sowie der entsprechenden Acetale 3,3-Dialkoxypropionitril bzw. 3,3-Dialkoxy-2-alkylpropionitril an, wobei der Anteil an C-alkyliertem Produkt bis zu 20 Gew.-% meistens bis zu 10 Gew.-% beträgt. Eine destillative Abtrennung des C-alkylierten Produktes aus dem Rohgemisch ist nur mit grossem Aufwand möglich. Es zeigte sich jedoch, dass dieses Rohgemisch mit einem Anteil von bis zu 20 Gew.-%, vorzugsweise von bis zu 10 Gew.-% an C-alkyliertem Produkt in gleicher Weise und mit gleich guter Ausbeute und Reinheit wie reines 3-Alkoxyacrylnitril (1) bzw. 3,3-Dialkoxypropionitril nach dem hier beschriebenen Verfahren Cytosin ergibt. Bevorzugte Ausgangsstoffe sind 3-Ethoxyacrylnitril, 3,3-Diethoxypropionitril oder Gemische hiervon oder Gemische dieser beiden Stoffe, die 3-Ethoxy-2-ethylacrylnitril und/oder 3,3-Diethoxy-2-ethylpropionitril in Mengen von 20 Gew.-%, vorzugsweise bis zu 10 Gew.-%, enthalten. Überraschend entsteht trotzdem reines Cytosin ohne Anteile von 5-Alkylcytosin, welches bei entsprechender Umsetzung von reinem 3-Alkoxy-2-alkylacrylnitril gebildet wird.

Zur Durchführung des erfindungsgemässen Verfahrens wird im allgemeinen so vorgegangen, dass eine Reaktionslösung, bestehend aus Lösungsmittel, Base, Harnstoff und 3-Alkoxyacrylnitril (1) bzw. 3,3-Dialkoxypropionitril (2) bzw. dem oben erwähnten Rohgemisch bei der Reaktionstemperatur zur Umsetzung gebracht, nach Reaktionsende neutralisiert, das Cytosin abfiltriert und gegebenenfalls durch Umkristallisation aus Wasser weiter gereinigt wird.

Als Lösungsmittel kommen polare Lösungsmittel, insbesondere ein- oder mehrwertige gerade- oder verzweigtkettige Alkohole mit 1 bis 12 Kohlenstoffatomen, vorzugsweise mit 1 bis 6 Kohlenstoffatomen oder Ätheralkohole wie Methylglykol oder polar aprotische Lösungsmittel in Frage. Als bevorzugte Lösungsmittel sind Ethanol, Propanol, Methylglykol, n- oder tert.-Butanol und n- oder tert.-Amylalkohol zu nennen, besonders verzweigte Alkohole wie tert.-Butanol.

Geeignete Basen sind Alkali- oder Erdalkalialkoholate oder Alkali- oder Erdalkalihydroxide, bevorzugt Alkalialkoholate der genannten Alkohole.

Bei Verwendung von Alkoholen als Lösungsmittel sollte der Alkoholatrest vorzugsweise gleich dem entsprechenden Alkohol sein, doch stellt dies keine Einschränkung dar. Das Molverhältnis von Base zu den Ausgangsstoffen (1) bzw. (2) beträgt 1 zu 1 bis 2 zu 1, vorzugsweise 1 zu 1 bis 1,5 zu 1.

Der Harnstoff wird in einer Menge von 1 bis 3, vorzugsweise 1 bis 2 mol per mol Nitrilgruppen der Ausgangsstoffe eingesetzt.

Die Reaktionstemperatur beträgt 40 bis 150 °C, vorzugsweise 60 bis 130 °C. Normaldruck bzw. der Eigendruck der Komponenten und gegebenenfalls Überdruck bis 10 bar ist möglich.

Die Reaktionsdauer beträgt in Abhängigkeit von der Reaktionstemperatur und der Zugabezeit etwa 0,5 bis 12 Stunden, die Zugabezeiten 0,5 bis 2 Stunden. Kurze Zugabe- und Reaktionszeiten werden ab etwa 70 °C erreicht.

Die Konzentration der Reaktionsteilnehmer richtet sich hauptsächlich nach ihrer Löslichkeit bzw. der Rührbarkeit der Reaktionslösung, im allgemeinen liegt die Anfangs-Alkoholat-Konzentration bei 0,5 bis 5 mol je Liter Lösung. Bei der Reaktion soll sorgfältige Rührung erfolgen.

Überraschend ist es erfindungsgemäss möglich, direkt aus der Reaktionslösung freies Cytosin zu erhalten. Es zeigte sich, dass der zunächst gebildete Niederschlag aus offenbar Alkali- bzw. Erdalkalisalzen des Cytosins bei Neutralisation mit Essigsäure, Ameisensäure, Chlorwasserstoff oder Phosphorsäure direkt in freies Cytosin überführt wurde. Dieses ist leicht gewinnbar, so dass die zahlreichen und aufwendigen Schritte bei der Aufarbeitung der Sulfate vermieden sind.

Bei der Aufarbeitung der Reaktionssätze zeigte es sich, dass zur Bildung des Cytosinringes eine Behandlung im sauren wässrigen Medium nicht notwendig ist, sondern dass das Cytosin allein durch Zugabe von Säuren oder Einstellung auf einen pH-Wert von etwa 7 aus den Metallsalzen freigesetzt wird.

Erfindungsgemäss werden Essigsäure, Ameisensäure, Chlorwasserstoff oder Phosphorsäure verwendet, wodurch es möglich ist, das organische Lösungsmittel ohne eine aufwendige Trocknungsstufe nach Abtrennung anorganischer Bestandteile wieder bei der Synthese einzusetzen.

Zur Neutralisation ist im allgemeinen die zur eingesetzten Base äquivalente Menge Säure notwendig.

Nach der Neutralisation fällt das Cytosin zusammen mit dem Alkali- oder Erdalkalisalz der Säure an, von dem es durch Behandlung mit Wasser leicht abgetrennt werden kann.

Die Aufarbeitung erfolgt bevorzugt unter wasserfreien Bedingungen, wobei die wasserfreie Säure zu der Reaktionslösung gegeben, das Cytosin zusammen mit dem Salz abfiltriert und das Lösungsmittel gegebenenfalls nach destillativer Reinigung wieder einsatzbereit zurückgewonnen wird. Es kann auch das Alkali- oder Erdalkalisalz des Cytosins abgetrennt und nach Auflösen in

Wasser durch Neutralisation mit einer Säure das Cytosin ausgefällt werden. Die organische Mutterlauge kann zur nächsten Umsetzung wieder eingesetzt werden, oder nach Zugabe einer wasserfreien Säure und Abtrennung vom Salz kann das Lösungsmittel wasserfrei zurückgewonnen werden.

Das erhaltene Cytosin kann durch Umkristallisation aus Wasser gegebenenfalls in Gegenwart von Aktivkohle in sehr hoher Reinheit erhalten werden.

Cytosin findet Verwendung unter anderem zur Herstellung von Chemotherapeutica, Agrochemikalien oder als Fotochemikalie.

Beispiel 1

Reaktion:

Zu einer Lösung von 58,8 g (0,525 mol) Kalium-tert-butylat in 300 ml tert.-Butanol werden 36 g (0,6 mol) Harnstoff und 58,8 g eines Gemisches aus 0,5 mol einer Nitrilkomponente, bestehend aus 3-Ethoxyacrylnitril, 3,3-Diethoxy-propionitril, 3-Ethoxy-2-ethylacrylnitril und 3,3-Diethoxy-2-ethylpropionitril, die zusammen 91,4 Gew.-% 3-Ethoxy-acrylnitril und 3,3-Diethoxypropionitril sowie zusammen 8,0 Gew.-% 3-Ethoxy-2-ethylacrylnitril und 3,3-Diethoxy-2-ethylpropionitril enthält, gegeben. Diese Lösung wird durch 3-stündiges Erhitzen auf Rückflusstemperatur zur Reaktion gebracht.

Aufarbeitung:

Nach dem Abkühlen auf etwa 40 °C wird mit 31,5 g (0,525 mol) Essigsäure neutralisiert (pH 7) und der gebildete Feststoff abfiltriert. Nach zweimaliger Umkristallisation des Feststoffes aus jeweils 250 ml Wasser mit 6 g Aktivkohle werden 29,8 g (53,6% d. Th.) reines Cytosin erhalten.

Das Lösungsmittel wird durch Destillation aufgearbeitet und kann wieder eingesetzt werden.

Beispiel 2

Zu einer Lösung von 58,8 g (0,525 mol) Kalium-tert-butylat in 300 ml tert.-Butanol werden 36 g Harnstoff und 71,5 g (0,5 mol) 3,3-Diethoxypropionitril gegeben und wie im Beispiel 1 die Lösung 3 Stunden zum Rückfluss erhitzt. Nach Aufarbeitung wie im Beispiel 1 werden 30,5 g (54,7% d. Th.) Cytosin erhalten.

Beispiel 3

Beispiel 2 wird wiederholt, jedoch mit 48,5 g (0,5 mol) 3-Ethoxyacrylnitril statt 3,3-Diethoxypropionitril. Es werden 30,4 g (54,7% d. Th.) Cytosin erhalten.

Beispiel 4

Zu einer Lösung von 42,1 g (0,5 mol) Kaliumethylat in 200 ml Ethanol werden 36 g (0,6 mol) Harnstoff und 58,8 g des Gemisches der Nitrile aus Beispiel 1 gegeben und darauf die Lösung 6 Stunden zum Rückfluss erhitzt. Nach Aufarbeitung wie im Beispiel 1 werden 22,1 g (39,8% d. Th.) Cytosin erhalten.

Beispiel 5

Die Reaktion des Beispiels 1 wird wiederholt. Zur Aufarbeitung wird nach dem Abkühlen der gebildete Feststoff abgetrennt, in 250 ml Wasser aufgenommen, durch Zugabe von 31,5 g (0,525 mol) Essigsäure neutralisiert und das ausgefällte Cytosin nach Zugabe von 6 g Aktivkohle umkristallisiert. Nach einer weiteren Umkristallisation aus 250 ml Wasser mit 6 g Aktivkohle werden 21,9 g (52,4% d. Th.) Cytosin erhalten.

Beispiel 6

Zu einer Suspension von 58,5 g (0,525 mol) Kalium-tert-butylat und 36 g (0,6 mol) Harnstoff in 300 ml tert.-Butanol werden bei 82 °C innerhalb von 2 Stunden 58,8 g des Gemisches der Nitrile aus Beispiel 1 zudosiert und anschliessend wird die Lösung noch 2 Stunden zum Rückfluss erhitzt. Nach Aufarbeitung wie im Beispiel 1 werden 34,6 g (62,3% d. Th.) Cytosin erhalten.

Beispiel 7

Die Reaktion des Beispiels 6 wird wiederholt, jedoch erfolgt die Zugabe der Nitrilkomponente bei der Reaktionstemperatur von 70 °C und die Reaktionszeit beträgt 12 Stunden. Nach Aufarbeitung wie im Beispiel 1 werden 34,3 g (61,8% d. Th.) Cytosin erhalten.

Beispiel 8

Zu einer Lösung von 58,8 g (0,525 mol) Kaliumn-butylat in 300 ml n-Butanol werden innerhalb von 2 Stunden bei 80 °C 58,8 g des Gemisches der Nitrile aus Beispiel 1 zudosiert und anschliessend wird die Lösung noch 2 Stunden bei 80 °C gerührt. Nach Aufarbeitung wie im Beispiel 1 werden 23,5 g (42,3% d. Th.) Cytosin erhalten.

**Patentansprüche**

1. Verfahren zur Herstellung von Cytosin, dadurch gekennzeichnet, dass man 3-Alkoxyacrylnitril (1) oder 3,3-Dialkoxypropionitril (2) oder Gemische hiervon, Harnstoff und Alkali- oder Erdalkalialkoholate bzw. -hydroxide als Base nach dem Reaktionsschema

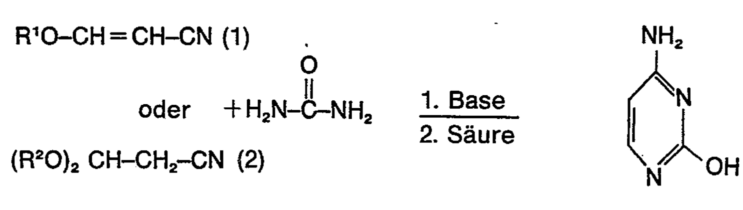


(1) oder (2) (3)

worin $R^1$ die Bedeutung geradkettige oder verzweigte Alkyl- oder Alkenylreste mit 1 bis 12 C-Atomen, isocyclische oder heterocyclische, einkernige oder mehrkernige aromatische oder cycloaliphatische Ringsysteme, welche gegebenenfalls inerte Substituenten tragen, oder $-(CH_2)_n$-Cyc mit Cyc = isocyclische oder heterocyclische einkernige oder mehrkernige aromatische oder cycloaliphatische Ringsysteme, welche gegebenenfalls inerte Substituenten an den Ringen tragen, und n = 0 bis 5 ist, die Reste $(CH_2)_mOR'$ oder $-(CH_2CH_2O)_pOR'$ mit m = 1 bis 5 und p = 1 bis 4 und R' = geradkettige oder verzweigte Alkylreste mit 1 bis 12 C-Atomen, und $R^2$ dieselbe Bedeutung wie $R^1$ oder die Bedeutung eines Alkylen oder Alkenylen-Restes eines Ringes mit 3 bis 6 Gliedern, der gegebenenfalls durch ein oder mehrere Heteroatome unterbrochen ist, hat, zur Reaktion bringt und zur Aufarbeitung nach beendeter Reaktion das Reaktionsgemisch durch Zugabe von Essigsäure, Ameisensäure, Chlorwasserstoff oder Phosphorsäure neutralisiert und freies Cytosin abscheidet und gewinnt.

2. Verfahren nach Anspruch 1, dadurch gekennzeichnet, dass das 3-Alkoxyacrylnitril (1) oder das 3,3-Dialkoxypropionitril (2) oder ein Gemisch hiervon als Verunreinigung Verbindungen der Formel

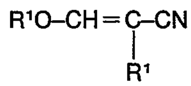 (3) oder 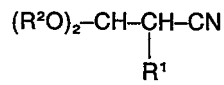 (4)

worin $R^1$ und $R^2$ dieselbe Bedeutung wie in der Formel (1) und (2) haben, in einer Menge von bis zu 20 Gew.-%, enthält.

3. Verfahren nach den Ansprüchen 1 oder 2, dadurch gekennzeichnet, dass die Nitril-Komponente zu der Lösung der Suspension aus Harnstoff und Alkoholat bzw. Hydroxid bei der Reaktionstemperatur zugegeben wird.

4. Verfahren nach einem der Ansprüche 1 bis 3, dadurch gekennzeichnet, dass als Lösungsmittel Alkohole mit 1 bis 12 C-Atomen verwendet werden.

## Claims

1. Process for the production of cytosine, characterised in that 3-alkoxyacrylonitrile (1) or 3,3-dialkoxypropionitrile (2) or mixtures thereof, urea and alkali or alkaline earth alcoholates or hydroxides as base are reacted according to the reaction scheme

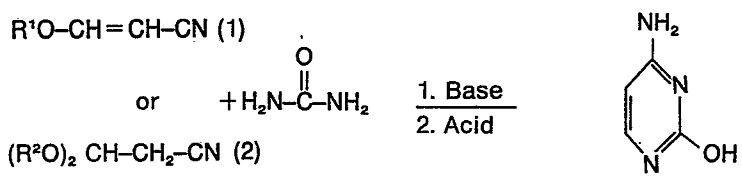

(3)

wherein $R^1$ has the meaning straight-chained or branched alkyl or alkenyl residues with 1 to 12 C-atoms, isocyclic or heterocyclic mononuclear or polynuclear aromatic or cycloaliphatic ring systems, which optionally carry inert substituents, or $-(CH_2)_n$ -Cyc with Cyc = isocyclic or heterocyclic mononuclear or polynuclear aromatic or cycloaliphatic ring systems, which optionally carry inert substituents on the rings, and n = 0 to 5, the residue $(CH_2)_mOR'$ or $-(CH_2CH_2O)_pOR'$ with m = 1 to 5 and p = 1 to 4 and R' = straight-chained or branched alkyl residues with 1 to 12 C-atoms, and $R^2$ has the same meaning as $R^1$ or the mean of an alkylene or alkenylene residue of a ring with 3 to 6 members, which is optionally interrupted by one or more hetero atomes, and, for working up at the end of the reaction, the reaction mixture is neutralised by additional acetic acid, formic acid, hydrogen chloride or phosphoric acid and free cytosine is separated and recovered.

2. Process according to claim 1, characterised in that the 3-alkoxyacrylonitrile (1) or the 3,3-dialkoxypropionitrile (2) or a mixture thereof contains, in an amount of up to 20% by weight, as impurity, compounds of the formulae

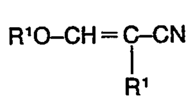 (3) or 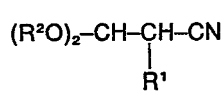 (4)

wherein $R^1$ and $R^2$ have the same meaning as in formula (1) and (2).

3. Process according to claim 1 or 2, characterised in that the nitrile component is added to the solution or suspension of urea and alcoholate or hydroxide at the reaction temperature.

4. Process according to one of claims 1 to 3, characterised in that alcohols with 1 to 12 C-atoms are used as solvent.

## Revendications

1. Procédé de préparation de la cytosine, caractérisé en ce qu'on fait réagir un 3-alcoxyacrylonitrile (1) ou un 3,3-dialcoxypropionitrile (2) ou leurs mélanges, l'urée et des alcoolates ou hydroxydes alcalins ou alcalinoterreux comme base, selon le schéma de réaction:

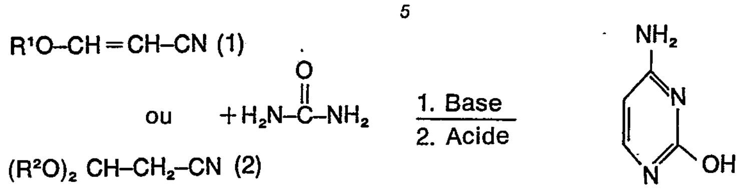


(3)

où $R^1$ représente des restes alkyles ou alcényles, linéaires ou ramifiés, ayant 1 à 12 atomes de carbone, des systèmes aromatiques ou cycloaliphatiques isocycliques ou hétérocycliques, monocycliques ou polycycliques, qui portent éventuellement des substituants inertes, ou bien $-(CH_2)_n$.Cyc, où Cyc représente des systèmes cycliques aromatiques ou cycloaliphatiques, isocycliques ou hétérocycliques, monocycliques ou polycycliques, qui portent éventuellement sur les noyaux des substituants inertes, et n vaut 0 à 5, des restes $(CH_2)_mOR'$ ou $-(CH_2CH_2O)_pOR'$, où m vaut 1 à 5 et p vaut 1 à 4 et R' représente des restes alkyles linéaires ou ramifiés ayant 1 à 12 atomes de carbone et $R^2$ a le même sens que $R^1$ ou représente un reste alkylène ou alcénylène d'un noyau comportant 3 à 6 chaînons, qui est éventuellement interrompu par un ou plusieurs hétéro-atomes et, après achèvement de la réaction, on neutralise le mélange réactionnel, pour son traitement d'élaboration, en lui ajoutant de l'acide acétique, de l'acide formique, de l'acide chlorhydrique ou de l'acide phosphorique, et l'on sépare et recueille la cytosine libre.

2. Procédé selon la revendication 1, caractérisé en ce que le 3-alcoxyacrylonitrile (1) ou le 3,3-dialcoxypropionitrile (2) ou un de leurs mélanges contient à titre d'impuretés, en une quantité pouvant aller jusqu'à 20% en poids, des composés de formules:

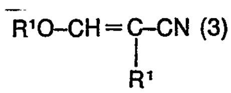

ou

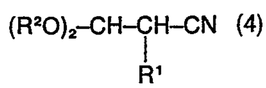

dans lesquelles $R^1$ et $R^2$ ont le même sens que pour les formules (1) et (2).

3. Procédé selon les revendications 1 ou 2, caractérisé en ce qu'on ajoute le composant nitrile à la solution ou suspension d'urée et d'alcoolate ou d'hydroxyde à la température de réaction.

4. Procédé selon l'une des revendications 1 à 3, caractérisé en ce qu'on utilise comme solvant des alcools ayant 1 à 12 atomes de carbone.